# EUROPEAN PATENT SPECIFICATION

(11) **EP 3 668 521 B1**
(45) Date of publication and mention of the grant of the patent: **02.10.2024**
(21) Application number: 18846442.4
(22) Date of filing: 13.08.2018
(51) Int. Cl.: C07K 5/02, A61K 38/00, A61K 9/00, A61P 3/10, A61P 19/02, A61P 19/06, A61P 25/28, A61P 27/10, A61P 35/00

(54) **INHIBITION OF OXIDATIVE STRESS, GLYCATION, AND PROTEIN CROSSLINKING**
HEMMUNG VON OXIDATIVEM STRESS, GLYKIERUNG UND PROTEINVERNETZUNG
INHIBITION DU STRESS OXYDANT, DE LA GLYCATION ET DE LA RÉTICULATION DE PROTÉINES

(30) Priority: 14.08.2017 US 201762545149 P
(43) Date of publication of application: 24.06.2020
(73) Proprietor: The Regents of the University of Colorado, a body corporate, Denver, CO 80203 (US)
(72) Inventor: NAGARAJ, Ram, Aurora Colorado 80016 (US); LINETSKY, Mikhail, Bratenahl Ohio 44108 (US); RAKETE, Stefan, 81373 Munich (DE); NAHOMI, Rooban, Aurora Colorado 80015 (US)
(74) Representative: Dehns
(86) International application number: PCT/US2018/046442
(87) International publication number: WO 2019/036336

(56) References cited:
- WO-A2-02/056804
- US-A- 4 419 343
- US-A- 4 766 133
- US-A- 5 869 456
- ORTWERTH B J ET AL: "Glutathione inhibits the glycation and crosslinking of lens proteins by ascorbic acid", EXPERIMENTAL EYE RESEARCH, ACADEMIC PRESS LTD, LONDON, vol. 47, no. 5, 1 November 1988 (1988-11-01), pages 737 - 750, XP022970366, ISSN: 0014-4835, [retrieved on 19881101], DOI: 10.1016/0014-4835(88)90041-3
- RAMAMURTHY B. ET AL: "Glutathione reverses early effects of glycation on myosin function", AMERICAN JOURNAL OF PHYSIOLOGY CELL PHYSIOLOGY, vol. 285, no. 2, 1 August 2003 (2003-08-01), US, pages C419 - C424, XP055790453, ISSN: 0363-6143, Retrieved from the Internet <URL:https://journals.physiology.org/doi/pdf/10.1152/ajpcell.00502.2002> DOI: 10.1152/ajpcell.00502.2002

## Description

### CROSS REFERENCE TO RELATED APPLICATION

This application claims the benefit of U.S. Provisional Patent Application Serial No. 62/545,149, filed August 14, 2017.

### STATEMENT OF GOVERNMENT INTEREST

This invention was made with government support under grant number EY023286 awarded by the National Institutes of Health. The government has certain rights in the invention.

### TECHNICAL FIELD

This disclosure relates to compositions used to treat or prevent glycation events and related pathologies in mammals. More particularly, this disclosure provides compositions having activities that inhibit glycation and protein crosslinking.

### BACKGROUND OF THE INVENTION

Glycation is the non-enzymatic reaction between reducing sugars, such as glucose, and proteins, lipids or nucleic acids. Since its first description by Maillard in 1912, the role of glycation in pathologies of the human body, including aging and diabetes, have been an active area of research. The formation of advanced glycation end products (AGEs) is a complicated molecular process involving simple and more complex multistep reactions, including the classical Maillard reaction, Schiff base formation, and Amadori product formation. Schiff bases and Amadori products are reversible reaction products, they can undergo oxidation, dehydration, polymerization and oxidative breakdown reactions to give rise to numerous structurally different stable products that are collectively known as AGEs. Oxygen, reactive oxygen species (ROS) and redox active transition metals accelerate AGE formation.

AGEs can be exogenously ingested (through food consumption) or be endogenously produced. Endogenous AGE formation is increased in diabetes, but AGEs are also formed at lower rates by normal metabolic processes. Environmental factors, such as diet and smoking influence the rate of AGE formation. Additionally, the levels of circulating AGEs are at least partially controlled by genetic factors.

The content of AGEs in a mammal is not only defined by the rate of their formation but also by the rate of their removal. Many cells have developed intrinsic detoxifying pathways that reduce the accumulation of AGEs. The glutathione-dependent glyoxalase system has a key role in the defense against glycation. This system uses reduced glutathione (GSH) to catalyze the conversion of glyoxal, methylglyoxal and other a-oxoaldehydes to the less toxic products, such as, D-lactate. Fructosamine kinases are expressed in various genomes including humans and destabilize Amadori products, leading to their spontaneous breakdown.

Some AGEs are benign, but others are more reactive, and are implicated in many age-related chronic diseases including diabetes mellitus, cardiovascular diseases, Alzheimer's disease, cancer, peripheral neuropathy, and other sensory losses such as deafness and blindness. The glycated hemoglobin level, also known as HbA1c, is determined and indicates the level of glycation occurring in the person. In recent years, the role of AGEs has been increasingly discussed in skin aging, and the potential of anti-AGE strategies has spawned the development of novel anti-aging cosmeceutical compounds.

Glycated substances are eliminated from the body slowly, since the renal clearance factor is only about 30%. This fact is used to provide a method of testing for sugar levels in diabetics. Red blood cells have a lifespan of 120 days and are easily accessible for measurement of recent increased presence of glycating product.

The long-term complications of diabetes include pathologies in the eye (cataractogenesis and retinopathy), kidney (nephropathy), neurons (neuropathy), and blood vessels (angiopathy and artherosclerosis). Glycation plays a role in all of these pathologies associated with diabetes.

Studies have shown that AGE may have a role in the development of atherosclerosis. Monocytes have AGE specific receptors (RAGE) and respond when stimulated by releasing cytokines. Minor injury to the blood vessel wall may expose sub-endothelial AGE, promote the infiltration of monocytes and initiate the development of atherosclerotic lesions. Circulating lipoproteins can also undergo glycation, which are then taken up by endothelial cells at a faster rate than non-glycated lipoprotein. Ramamurthy et al., American Jouirnal of Physiology-Cell Physiology, 2003, 285(2), C419-C424, suggest that glutathione, in addition to its antioxidant function, could play an important role in preventing the progress of glycation of intracellular proteins.

As a person ages the minimum distance from the eye at which an object will come into focus increases. This loss in the ability to focus in nearby objects is called presbyopia. The highest incidence of onset of presbyopia occurs in people ages 42-44. Presbyopia occurs because as a person ages the eye's accommodative ability which uses near reflex-pupil constriction, convergence of the eyes and particularly ciliary muscle contraction, decreases, but the major reason for presbyopia is crosslinking and aggregation of lens proteins. Orthwert et al., Experimental Eye Research, 1988, 47(5), 737-750, found that high levels of glutathione present in normal lens tissue would be sufficient to ensure that glycation and crosslinking would not be a significant reaction in normal lens tissue.

This reduction in lens accommodation results in an inadequate change in the normal thickening and increased curvature of the anterior surface of the lens that is necessary for the shift in focus from distant objects to near objects. Presbyopia is a normal and inevitable effect of aging and more than 1 billion people worldwide were presbyopic in 2005.

Thus, compositions that can counteract the long-term effects of AGE formation and prevent or treat AGE-related pathologies are needed.

### SUMMARY

Glycation-related conditions comprise mammalian diseases or disorders that are due to, or related to, the presence of glycated proteins, lipids, and nucleic acids, and include, but are not limited to, inflammatory responses, atherosclerosis, Alzheimer's disease, diabetes, eye disorders, cancer, cardiovascular diseases, and other AGE-related conditions.

This disclosure provides compositions for reducing advanced glycation end products (AGEs) in a mammalian tissue and therapeutic methods of treating and preventing AGE-associated diseases and disorders. The compositions of this disclosure comprise glutathione diethyl ester thioethylguanidine disulfide (GTG) that has the chemical structure: 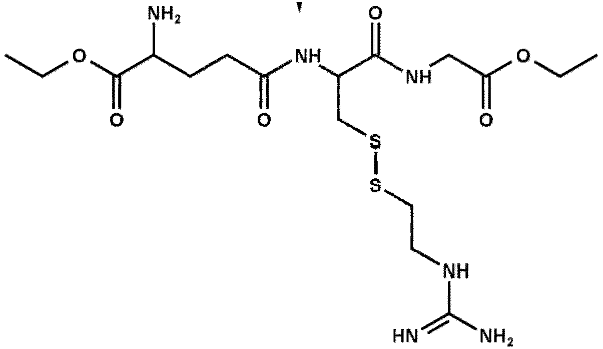 as well as pharmaceutically acceptable salts, stereoisomers, and metabolites thereof. These compositions may be formulated as a tablet or capsule for oral administration, or as a sterile liquid for parenteral administration, or as a liquid for ophthalmic administration. The composition may be a mono-phasic pharmaceutical composition suitable for parenteral or oral administration consisting essentially of a therapeutically-effective amount of the GTG compounds of this disclosure, and a pharmaceutically acceptable excipient. These compositions may also be in the form of a beverage or foodstuff comprising the GTG compounds of this disclosure.

Therapeutic methods of this disclosure include treating or preventing adverse health consequences of oxidative or carbonyl stress, or glycation-related conditions by administering to a mammalian subject an effective amount of an anti-glycation composition comprising a GTG compound of this disclosure, as described above. The glycation-related conditions that are effectively addressed by these methods include the formation of glycation end products, AGE formation, glycation reactions of proteins, lipids and/or nucleic acids, aging effects related to glycation reactions, cancer, diabetes, and complications of diabetes (Type I and II), rheumatoid arthritis, Alzheimer's disease, uremia, neurotoxicity, atherosclerosis, inflammatory reactions, ventricular hypertrophy, angiopathy, myocarditis, nephritis, arthritis, glomerulonephritis, microangiopathies, and renal insufficiency. These therapeutic methods are preferably applied to a human.

This disclosure also provides for the use of a GTG compound of this disclosure, in the manufacture of a medicament for the treatment, prevention, or amelioration of glycation-related conditions. Additionally, this disclosure provides a GTG compound of this disclosure for use in the treatment of glycation-related conditions.

This Summary is neither intended nor should it be construed as representative of the full extent and scope of the present disclosure. Moreover, references made herein to "the present disclosure," or aspects thereof, should be understood to mean certain embodiments of the present disclosure and should not necessarily be construed as limiting all embodiments to a particular description. The present disclosure is set forth in various levels of detail in this Summary as well as in the attached drawings and the Description of Embodiments and no limitation as to the scope of the present disclosure is intended by either the inclusion or non-inclusion of elements, components, etc. in this Summary. Additional aspects of the technology will become readily apparent from the Description of Embodiments, particularly when taken together with the drawings.

### DESCRIPTION OF FIGURES

The following drawings form part of the present disclosure and are included to further demonstrate certain aspects of the present technology. The embodiments may be better understood by reference to one or more of these drawings in combination with the detailed description presented herein.
**FIG.** 1 is a schematic representation of the synthesis scheme for glutathione diethyl ester thioethylguanidine disulfide (GTG).
**FIGS. 2A-2D** show the results of stability testing of GTG in various buffers and conditions: **FIG. 2A** is results in 0.1% formic acid at three temperatures; **FIG. 2B** is results in 0.1 M phosphate buffer, pH 5, at three temperatures; **FIG.2C** is results in phosphate buffer pH 7; **FIG. 2D** is results in 0.1 M phosphate buffer pH 9.
**FIG.** 3 depicts the dominant degradation pathway of GTG by the loss of either one or two molecules of ethanol from both terminal carboxyl groups.

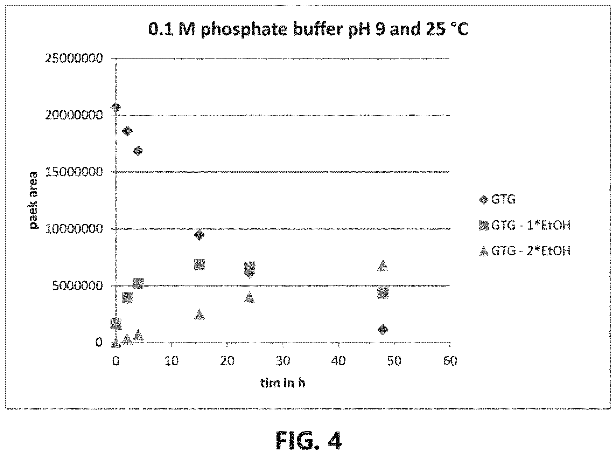

**FIG.** 4 shows the results of UPLC-MS analysis of degradation products of GTG.

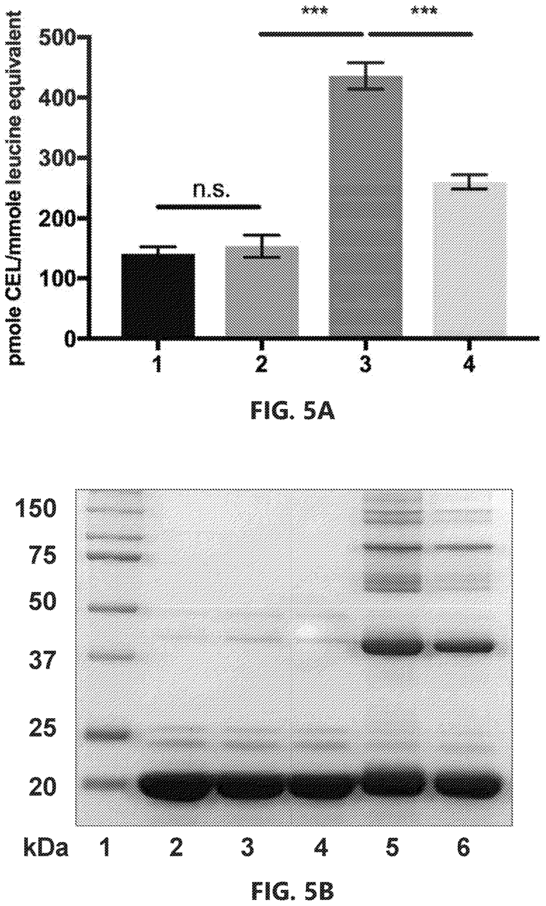

**FIG. 5A** shows the Nε-carboxylethyllysine (CEL) levels in αB-crystallin incubated with GTG and MGO after acid hydrolysis. Legend: 1. αB-crystallin control, 2. αB-crystallin + 250 µM GTG, 3. αB-crystallin + 250 µM MGO, 4. αB-crystallin + 250 µM GTG + 250 µM MGO. The bar graphs are means ± SD of triplicate measurements. *** p<0.0005. **FIG. 5B** shows SDS-PAGE (12 % gel, 7.5 µg protein, Coomassie staining) analyses of αB-crystallin treated with MGO and GTG. Lanes: 1. MW marker, 2. αB-crystallin standard, 3. αB-crystallin blank, 4. αB-crystallin + 250 µM GTG, 5. αB-crystallin + 250 µM MGO, 5. αB-crystallin + 250 µM GTG and 250 µM MGO.

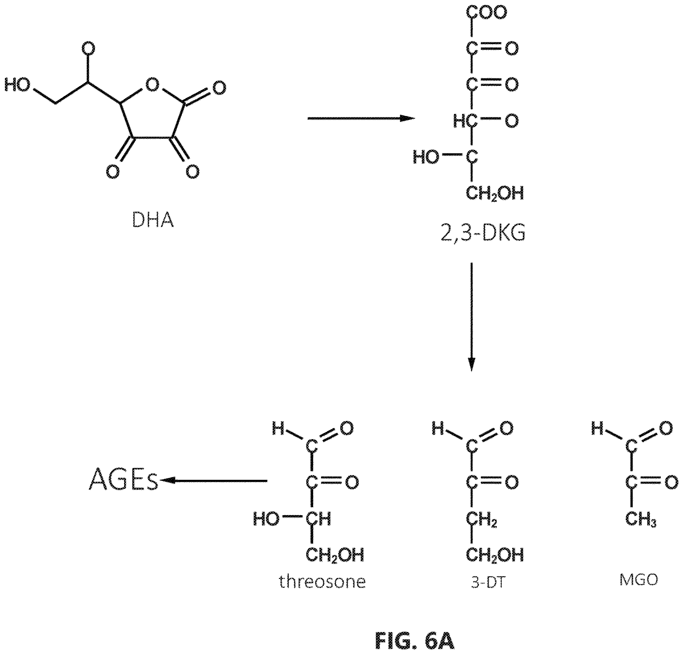

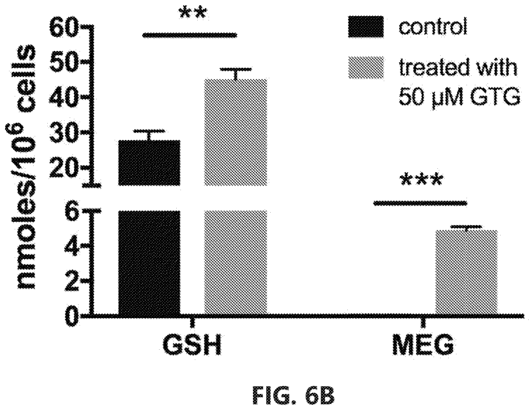

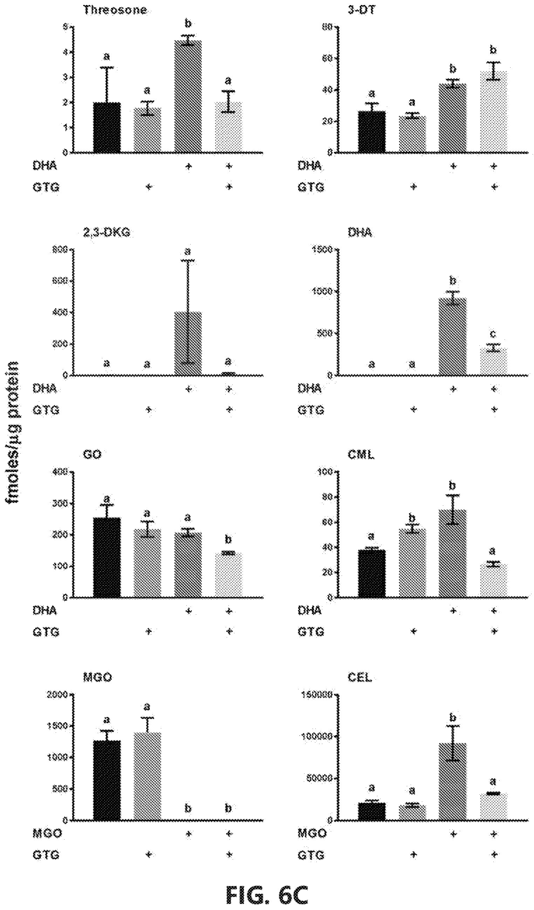

**FIG. 6A** depicts the scheme of dehydroascorbate (DHA) degradation and formation of reactive α-dicarbonyls and AGEs. **FIG. 6B** shows the cellular levels of GSH and mercaptoethylguanidine in FHL 124 cells after treatment with 50 mM GTG for 2h. The bar graphs are means ± SD of triplicate measurements. ** p<0.005, *** p<0.0005. **FIG. 6C** shows the cellular levels of α-dicarbonyls (threosone, 3-DT, 2,3-DKG, DHA, GO, MGO) and AGEs (CML & CEL) in FHL124 cells pretreated with GTG before either DHA or MGO was added. Bars that do not share a common superscript are statistically different (P<0.05).
**FIG.** 7 shows AGE levels in lenses and retinas from diabetic mice following GTG (1 nmole/g body weight) injection intraperitoneally once every two days. The bar graphs are the means ± SD of at least 8 measurements. *p<0.05, **p< 0.005.
**FIGS. 8A** and **8B** show that GTG can be delivered to lens nucleus. LC-MS/MS analysis of lens fractions (cortex to nucleus) for GSH **(****FIG. 8A****)** and MEG **(****FIG. 8B****).** The bar graphs indicate the mean ± SD of triplicate measurements. NS, not significant; *p<0.05; **p<0.005.
**FIGS. 9A** and **9B** show that GTG reduces lens stiffness. FIG. 9A shows axial compressive strain and FIG. 9B shows equatorial compressive strain plotted against a fixed applied load for mouse lenses treated without or with GTG. The bar graphs indicate the means ± SD of triplicate measurements. *p<0.005.

### DESCRIPTION OF EMBODIMENTS

The present disclosure is drawn to compositions that reduce advanced glycation end product (AGE) accumulation in mammalian tissues. These compositions distribute into tissues of the eye for the treatment of patients with or at risk of developing glycation-related conditions.

To facilitate an understanding of the embodiments presented, the following explanations are provided.

The singular terms "a," "an," and "the" include plural referents unless context clearly indicates otherwise. Similarly, the word "or" is intended to include "and" unless the context clearly indicates otherwise. The term "comprises" means "includes." Also, "comprising A or B" means including A or B, or A and B, unless the context clearly indicates otherwise. It is to be further understood that all molecular weight or molecular mass values given for compounds are approximate, and are provided for description. Although methods and materials similar or equivalent to those described herein can be used in the practice or testing of this disclosure, suitable methods and materials are described below. In addition, the materials, methods, and examples are illustrative only and not intended to be limiting.

"Administration of" and "administering a" compound, composition, or agent should be understood to mean providing a compound, composition, or agent, a prodrug of a compound, composition, or agent, or a pharmaceutical composition as described herein. The compound, agent or composition can be provided or administered by another person to the subject (e.g., intravenously) or it can be self-administered by the subject (e.g., orally as tablets, capsules, supplements, or medical foods).

The term "subject" refers to animals, including mammals (for example, humans and veterinary animals such as dogs, cats, pigs, horses, sheep, and cattle).

"Pharmaceutical compositions" are compositions that include an amount (for example, a unit dosage) of one or more of the disclosed compounds together with one or more non-toxic pharmaceutically acceptable additives, including carriers, diluents, and/or adjuvants, and optionally other biologically active ingredients. Such pharmaceutical compositions can be prepared by standard pharmaceutical formulation techniques such as those disclosed in Remington's Pharmaceutical Sciences, Mack Publishing Co., Easton, Pa. (19th Edition).

The terms "pharmaceutically acceptable salt or ester" refers to salts or esters prepared by conventional means that include salts, e.g., of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid, and the like.

"Pharmaceutically acceptable salts" of the presently disclosed compounds also include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. These salts may be prepared by standard procedures, for example by reacting the free acid with a suitable organic or inorganic base. Any chemical compound recited in this specification may alternatively be administered as a pharmaceutically acceptable salt thereof. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). When compounds disclosed herein include an acidic function such as a carboxy group, then suitable pharmaceutically acceptable cation pairs for the carboxy group are well known to those skilled in the art and include alkaline, alkaline earth, ammonium, quaternary ammonium cations and the like. Such salts are known to those of skill in the art. For additional examples of "pharmacologically acceptable salts," see Berge et al., J. Pharm. Sci. 66:1 (1977).

A "therapeutically effective amount" of the disclosed compounds or compositions is a dosage that is sufficient to achieve a desired therapeutic effect, such as inhibition of AGE formation or accumulation in a mammalian tissue or an anti-AGE or anti-oxidative effect. A therapeutically effective amount may be an amount sufficient to achieve tissue concentrations at the site of action that are similar to those that are shown to modulate AGE formation or AGE-related adverse health effects in tissue culture, in vitro, or in vivo. For example, a therapeutically effective amount of a GTG compound of this disclosure may be such that the subject receives a dosage of about 0.1 µg/kg body weight/day to about 1000 mg/kg body weight/day, for example, a dosage of about 1 µg/kg body weight/day to about 1000 µg/kg body weight/day, such as a dosage of about 5 µg/kg body weight/day to about 500 µg/kg body weight/day.

The term "stereoisomer" refers to a molecule that is an enantiomer, diasteromer or geometric isomer of a molecule. Stereoisomers, unlike structural isomers, do not differ with respect to the number and types of atoms in the molecule's structure but with respect to the spatial arrangement of the molecule's atoms. Examples of stereoisomers include the (+) and (-) forms of optically active molecules.

The term "modulate" refers to the ability of a GTG compound of this disclosure to alter the amount, degree, or rate of a biological function, the progression of a disease, or amelioration of a condition. For example, modulating can refer to the ability of a compound to elicit an increase or decrease in AGE formation or accumulation in a mammalian tissue, or to inhibit or prevent a glycation-related condition in a mammalian subject.

"Treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. The phrase "treating a disease" is inclusive of inhibiting the full development of a disease or condition, for example, in a subject who is at risk for a disease, or who has a disease, such as cancer or a disease associated with a compromised immune system. "Preventing" a disease or condition refers to prophylactically administering a composition to a subject who does not exhibit signs of a disease or exhibits only early signs of the disease, for the purpose of decreasing the risk of developing a pathology or condition, or diminishing the severity of a pathology or condition. The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

The term "prodrug" also is intended to include any covalently bonded carriers that release an active parent anti-glycation compound of this disclosure in vivo when the prodrug is administered to a subject. Because prodrugs often have enhanced properties relative to the active agent pharmaceutical, such as solubility and bioavailability, the compounds disclosed herein can be delivered in prodrug form. Thus, also contemplated are prodrugs of the presently disclosed GTG compounds, methods of delivering prodrugs, and compositions containing such prodrugs. Prodrugs of the disclosed compounds typically are prepared by modifying one or more functional groups present in the compound in such a way that the modifications are cleaved, either in routine manipulation or in vivo, to yield the parent compound. Prodrugs include compounds having a phosphonate and/or amino group functionalized with any group that is cleaved in vivo to yield the corresponding amino and/or phosphonate group, respectively. Examples of prodrugs include, without limitation, compounds having an acylated amino group and/or a phosphonate ester or phosphonate amide group.

### Compounds

This disclosure provides compositions and compositions for use in methods that treat or prevent glycation-related conditions in mammalian subjects and is based on the inventors' surprising discovery that glutathione diethyl ester thioethylguanidine disulfide (GTG) is able to penetrate the plasma membrane of mammalian cells after which it is immediately reduced intracellularly and hydrolyzed to glutathione and mercaptoethylguanidine. The therapeutically useful GTG compound of this disclosure is glutathione diethyl ester thioethylguanidine disulfide (GTG) that has the chemical structure: 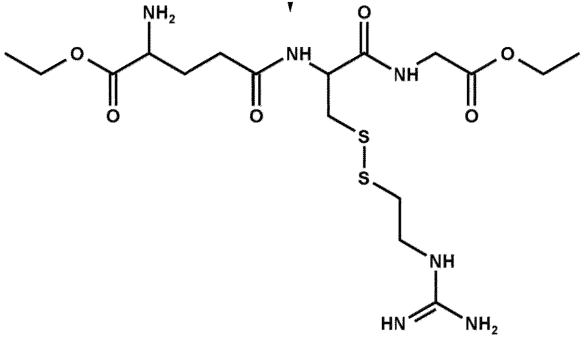 as well as pharmaceutically acceptable salts, stereoisomers, and tautomers thereof. These compounds may be prepared as described in Example 1 of this disclosure.

For therapeutic use, salts of the GTG compounds of this disclosure are those wherein the counter-ion is pharmaceutically acceptable. However, salts of acids and bases which are non-pharmaceutically acceptable may also find use, for example, in the preparation or purification of a pharmaceutically acceptable compound.

The pharmaceutically acceptable acid and base addition salts are meant to comprise the therapeutically active non-toxic acid and base addition salt forms which the disclosed GTG compounds can form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic, and like acids. Conversely said salt forms can be converted by treatment with an appropriate base into the free base form.

The compounds containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine, and the like.

The GTG compounds of this disclosure include one or more asymmetric centers. Thus, these compounds can exist in different stereoisomeric forms. Accordingly, GTG compounds and compositions of this disclosure may be provided as individual pure enantiomers or as stereoisomeric mixtures, including racemic mixtures. The compounds disclosed herein may be synthesized in, or are purified to be in, substantially enantiopure form, such as in a 90% enantiomeric excess, a 95% enantiomeric excess, a 97% enantiomeric excess or even in greater than a 99% enantiomeric excess, such as in enantiopure form. Some of the GTG compounds described herein may also exist in their tautomeric form.

### Compositions

Compositions comprising the anti-glycation glutathione diethyl ester thioethylguanidine disulfide (GTG) compounds of this disclosure with low cytotoxicity and good bioavailability (including blood-brain barrier penetration) are contemplated. The compositions of this disclosure may be formed by combining the anti-glycation GTG compounds of this disclosure with pharmaceutically acceptable excipients, and optionally sustained-release matrices, such as biodegradable polymers, to form therapeutic compositions. Therefore, also disclosed are pharmaceutical compositions including one or more of any of the GTG compounds disclosed above and a pharmaceutically acceptable carrier. The composition may comprise a unit dosage form of the composition, and may further comprise instructions for administering the composition to a subject, for example, instructions for administering the composition to achieve an anti-glycation therapeutic effect or to inhibit the formation or progression of a glycation-related disease or disorder. Such pharmaceutical compositions may be used in methods for treating or preventing glycation-related disease or disorder in a subject by administering to the subject a therapeutically effective amount of the composition.

These compositions can be in the form of tablets, capsules, powders, granules, lozenges, liquid or gel preparations, such as oral, topical, or sterile parenteral solutions or suspensions (e.g., eye or ear drops, throat or nasal sprays, etc.), transdermal patches, and other forms known in the art.

These compositions can be administered systemically or locally in any manner appropriate to the treatment of a given condition, including orally, parenterally, intrathecally, rectally, nasally, buccally, vaginally, topically, optically, by inhalation spray, or via an implanted reservoir. The term "parenterally" as used herein includes, but is not limited to, subcutaneous, intravenous, intramuscular, intrasternal, intrasynovial, intrathecal, intrahepatic, intralesional, and intracranial administration, for example, by injection or infusion. For treatment of the central nervous system, these compositions may readily penetrate the blood-brain barrier when peripherally or intraventricularly administered.

Acceptable carriers for use in these compositions include, but are not limited to, ion exchangers, alumina, aluminum stearate, lecithin, serum proteins (such as human serum albumin), buffers (such as phosphates), glycine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

Tablets and capsules for oral administration can be in a form suitable for unit dose presentation and can contain conventional pharmaceutically acceptable excipients. Examples of these include binding agents such as syrup, acacia, gelatin, sorbitol, tragacanth, and polyvinylpyrrolidone; fillers such as lactose, sugar, corn starch, calcium phosphate, sorbitol, or glycine; tableting lubricants, such as magnesium stearate, talc, polyethylene glycol, or silica; disintegrants, such as potato starch; and dispersing or wetting agents, such as sodium lauryl sulfate. Oral liquid preparations can be in the form of, for example, aqueous or oily suspensions, solutions, emulsions, syrups or elixirs, or can be presented as a dry product for reconstitution with water or other suitable vehicle before use.

Drops, such as eye drops (ophthalmological compositions) or nose drops, may be formulated with an aqueous or nonaqueous base also comprising one or more dispersing agents, solubilizing agents or suspending agents. Liquid sprays are conveniently delivered from pressurized packs. Drops can be delivered by means of a simple eye dropper-capped bottle or by means of a plastic bottle adapted to deliver liquid contents dropwise by means of a specially shaped closure.

These compositions can also be administered parenterally in a sterile aqueous or oleaginous medium. The composition can be dissolved or suspended in a non-toxic parenterally-acceptable diluent or solvent, e.g., as a solution in 1,3-butanediol. Commonly used vehicles and solvents include water, physiological saline, Hank's solution, Ringer's solution, and sterile, fixed oils, including synthetic mono- or di-glycerides, etc. For topical application, the drug may be made up into a solution, suspension, cream, lotion, or ointment in a suitable aqueous or non-aqueous vehicle. Additives may also include, for example, buffers such as sodium metabisulphite or disodium edeate; preservatives such as bactericidal and fungicidal agents, including phenyl mercuric acetate or nitrate, benzalkonium chloride or chlorhexidine, and thickening agents, such as hypromellose.

The dosage unit involved depends, for example, on the condition treated, nature of the formulation, nature of the condition, embodiment of the claimed pharmaceutical compositions, mode of administration, and condition and weight of the patient. Dosage levels are typically sufficient to achieve a tissue concentration at the site of action that is at least the same as a concentration that has been shown to be active in vitro, in vivo, or in tissue culture. For example, a dosage of about 0.1 µg/kg body weight/day to about 1000 mg/kg body weight/day, for example, a dosage of about 1 µg/kg body weight/day to about 1000 µg/kg body weight/day, such as a dosage of about 5 µg/kg body weight/day to about 500 µg/kg body weight/day can be useful for treatment of a particular condition. Thus, these compositions may comprise about 0.0001 g to about 1000 g of the anti-glycation GTG compounds of this disclosure, as described above, added to a composition comprising the GTG compound in an amount of about 0.0001 g to about 1000 g.

The GTG compounds of this disclosure for prevention or treatment of glycation-related conditions, or inhibition of glycation products and AGE, may also be prepared and provided within a beverage or foodstuff. Such compositions may include foodstuffs, food products, nutritive and non-nutritive sweeteners, pharmaceutical, nutraceutical, or dietary supplement formulations. The compositions of this disclosure may also function as additives to foods, to be combined with food products, including foods wherein a GTG compound of this disclosure can be added to provide anti-glycation activity to the food. Thus, anti-glycation compositions for the prevention or treatment of glycation-related conditions may comprise ready-to-eat-cereals, fruit juices, candies, chewing gum, nutritional supplements, enhanced water beverages, carbonated and non-carbonated drinks, alcoholic beverages such as beer and wine, baby food, and many other foodstuffs and beverages. The anti-glycation compositions of the present invention may be used an animal feed additive.

### Diseases Addressed by the Therapeutic Methods

The GTG compounds of this disclosure block both oxidative and carbonyl stress. Because oxidative and carbonyl stress are two fundamental drivers of pathologies in many diseases, these GTG compounds may be effective in the prevention or treatment of diseases and disorders including cancer, arthritis, diabetes, complications of the eye, atherosclerosis, and neurological diseases.

Anti-glycation activity, or glycation inhibitory activity, are terms which refer to the ability of a compound or composition to interfere with glycation reactions such that the reaction of amino groups of proteins, lipids, or nucleic acids with sugar aldehyde or keto groups to produce modified amino groups, are altered or prevented, or the formation of advanced glycation end-products are altered or prevented, in vivo or in vitro. Anti-glycation activity may also include increased destruction and elimination of advanced glycation end-products. AGEs form at a constant but slow rate in the normal body, starting in early embryonic development, and accumulate with time. However, their formation is markedly accelerated with aging or with increased exposure to the sugar sources, such as glucose, fructose, and galactose.

AGEs are important pathogenetic mediators of almost all diabetes complications, conventionally grouped into micro- or macro-angiopathies. For instance, AGEs are found in retinal blood vessels of diabetic patients, and their levels correlate with those in serum, as well as with severity of retinopathy. AGEs are implicated in a vast array of age-related degradations from coronary disease to skin aging. Some AGEs are benign, but others are more reactive than the sugars they are derived from, and are implicated in many age-related chronic diseases, such as diabetes mellitus (in which beta cells are damaged), cardiovascular diseases (in which the endothelium, fibrinogen, and collagen are damaged), Alzheimer's disease (wherein amyloid proteins are side products of the reactions progressing to AGEs), cancer (including release of acrylamide and other side products), peripheral neuropathy and other sensory losses such as deafness due to demyelination, renal dysfunction and blindness (which occur because of microvascular damage). This range of diseases and disorders shows the extent of the effects of AGEs interfering with molecular and cellular functioning throughout the body and the other effects associated with AGEs, such as the release of highly-oxidizing side products such as hydrogen peroxide. Glycated substances are eliminated from the body slowly, and the renal clearance factor is only about 30%. As a consequence, long-lived cells (such as neurons), long-lasting proteins (such as eye crystallins and collagen), and DNA may accumulate substantial damage over time. Metabolically-active cells such as those in kidney's glomeruli, retinal cells in the eyes, and insulin-producing beta cells in the pancreas, are also at high risk of damage. The endothelial cells of the blood vessels are damaged directly by glycation, which has implications in atherosclerosis. Atherosclerotic plaque tends to accumulate at areas of high blood flow due to the increased presentation of sugar molecules, and glycation end-products at these points. Damage by glycation results in stiffening of the collagens in the blood vessel walls, and contributes to high blood pressure. Glycation also causes weakening of blood vessel walls, which may lead to micro-or macro-aneurisms, which in turn, if formed in the brain, may lead to strokes.

In addition to endogenously formed AGE, AGEs can also be introduced in the body from exogenous sources. Exogenous AGE may be consumed as "dietary" or "preformed" AGEs. These are formed when sugars are cooked with proteins or fats. Tobacco smoke, for example, is a well-known exogenous source of AGEs. The combustion of various pre-AGEs in tobacco during smoking gives rise to reactive and toxic AGEs. Serum AGEs or LDL-linked AGEs are significantly elevated in cigarette smokers. Diabetic smokers, as a result, are reported to exhibit greater AGE deposition in their arteries and ocular lenses.

Specific diseases or disorders for which the therapeutic methods of this disclosure are beneficial include, but are not limited to, glycation-related conditions including formation or accumulation of glycation end products, AGE formation, glycation reactions of proteins, lipids and/or nucleic acids, aging effects related to glycation reactions, cancer, diabetes, complications of diabetes (Type I and II), rheumatoid arthritis, neurological diseases and disorders including Alzheimer's disease, uremia, neurotoxicity, atherosclerosis, inflammatory reactions, ventricular hypertrophy, angiopathy, myocarditis, nephritis, arthritis, glomerulonephritis, microangiopathies, renal insufficiency, and presbyopia.

### Therapeutic Methods

As noted above, the GTG compounds of this disclosure can be used in the treatment or prevention of glycation-related conditions, including methods of treating or preventing complications and pathologies of conditions related to the production and/or presence of glycated molecules such as glycated proteins, lipids, and/or nucleic acids in a mammalian subject. These methods include the administration of an effective amount of a GTG-containing anti-glycation composition of this disclosure to a mammalian subject to prevent or reduce glycation events occurring in the subject or to treat or prevent glycation-related conditions in the subject.

Atherosclerosis is significantly accelerated in diabetic patients and is associated with greater risk of cardiovascular and cerebrovascular mortality. Preclinical and clinical studies have shown that AGEs play a significant role in the formation and progression of atherosclerotic lesions. Increased AGE accumulation in the diabetic vascular tissues has been associated with changes in endothelial cell, macrophage, and smooth muscle cell function. In addition, AGEs can modify LDL cholesterol in such a way that it tends to become easily oxidized and deposited within vessel walls, causing streak formation and, in time, atheroma. AGE-crosslink formation results in arterial stiffening with loss of elasticity of large vessels.

As described above, the inventors have surprisingly discovered that GTG compounds of this disclosure are able to penetrate the plasma membrane of mammalian cells followed by immediate intracellular reduction and hydrolysis to glutathione and mercaptoethylguanidine. Thus, without intending to be bound by theory, the anti-glycation compounds and compositions of this disclosure block both oxidative and carbonyl stress and are believed to significantly reduce the level or production of AGEs and/or significantly enhance the elimination of AGEs within a mammalian subject following administration to the subject.

Methods of this disclosure therefore include inhibiting the formation of glycation end products, inhibition of AGE formation, inhibition of glycation reactions of proteins, lipids and/or nucleic acids, inhibition of aging effects related to glycation reaction, and methods for treatment or prevention of glycation-related conditions including, but not limited to, complications of diabetes (Type I and II), rheumatoid arthritis, Alzheimer's disease, uremia, neurotoxicity, atherosclerosis, inflammatory reactions, ventricular hypertrophy, angiopathy, myocarditis, nephritis, arthritis, glomerulonephritis, microangiopathies, and renal insufficiency, or methods of preventing accumulation of glycation products. Methods for inhibiting glycation or inhibiting AGE formation, prevention or treatment of glycation-related conditions comprise administering or providing an effective amount of an anti-glycation composition comprising a GTG compound of this disclosure to a mammalian subject, wherein the anti-glycation composition alters the effects of glycation, glycation activity, glycation rate or amount of glycation products such as AGE in the subject. An amount of glycation activity may be measured by an A1C test of glycated hemoglobin. A reduction in the A1C measurement indicates a lower amount of glycation activity in the body. Measurements for glycated proteins are known to those skilled in the art as described in U.S. Patent No. 5,506,144.

Methods of this disclosure comprise making and using anti-glycation compositions. Such compositions may be consumed by healthy young and adult animals and humans, as well as humans or animals at risk for developing, or suffering from, diabetes, atherosclerosis or similar glycation-related conditions. Food, beverages, and nutritional supplement anti-glycation compositions may be used to provide anti-glycation activity to a subject and provide a benefit in lowering the potential cross-linking of protein and sugars to promote health and wellness and reduce the incidence of glycation-related diseases or disorders forming in a mammalian subject.

In the methods of this disclosure for prevention or treatment of glycation-related conditions, or inhibition of glycation products and AGE, for example, a beverage or foodstuff comprising an anti-glycation GTG compound of this disclosure may be ingested by mammalian subjects, to reduce the effects of glycation.

Methods of preventing, treating, or ameliorating glycation-related conditions, or inhibition of the production of glycation products and AGE, include providing an effective amount of an anti-glycation composition of this disclosure for protecting or enhancing cognitive function. Cognition is a general term covering many aspects of brain function, including learning, memory, thinking and reasoning. These processes may decline during the natural aging process or in the event of degenerative disease. Advanced glycation end products and free radical damage may be a natural part of aging, leading to reduced cognitive function and motor skills, and may lead to accelerated forms of dementia, Alzheimer's or Parkinson's disease. For example, a beverage or foodstuff, or composition comprising an anti-glycation GTG compound of this disclosure may be ingested by mammalian subjects to reduce the effects of glycation on the nervous system, reduce inflammatory reactions, and prevent damage to the circulatory system. Such anti-glycation compositions may be used to protect against cognitive degradation, restore optimal cognitive functionality, enhance cognitive performance, promote healthy brain function, aid in combating oxidative-induced cognitive degradation, strengthen cognitive function defense, and to stimulate coherent cognitive processes.

Methods of preventing, treating, or ameliorating glycation-related conditions, or inhibition of the production of glycation products and AGE, include providing an effective amount of an anti-glycation composition comprising a GTG composition of this disclosure for vision. Major risk factors in vision loss, cataracts, and diabetic retinopathy are the hardening of the lens and capillaries and retina in the eyes. It is well established that diabetics suffer a much higher incidence of vision impairment relative to the general population, in part as a result of AGE accumulation in the eye. For example, a beverage or foodstuff comprising an anti-glycation composition may be ingested by diabetic mammalian subjects to reduce the effects of glycation on the eye and associated structures, or ophthalmological compositions comprising GTG may be administered for the treatment or reducing the progression of presbyopia in older or middle-aged subjects. Such compositions are useful for promoting vision wellness, defending against age-related vision degradation, restoring eye capillary circulation, protecting against age-related vision impairment, protecting against glycation-induced vision impairment, reducing age-related vision impairment, promoting health of the eyes, encouraging lens clarity, sustaining healthy vision, and treating presbyopia by improving depth of focus in patients with presbyopia.

This disclosure is further illustrated by the following examples, which are not to be construed in any way as imposing limitations upon the scope

### EXAMPLES

### Example 1

### Synthesis of Glutathione Diethyl Ester Thioethylguanidine Disulfide

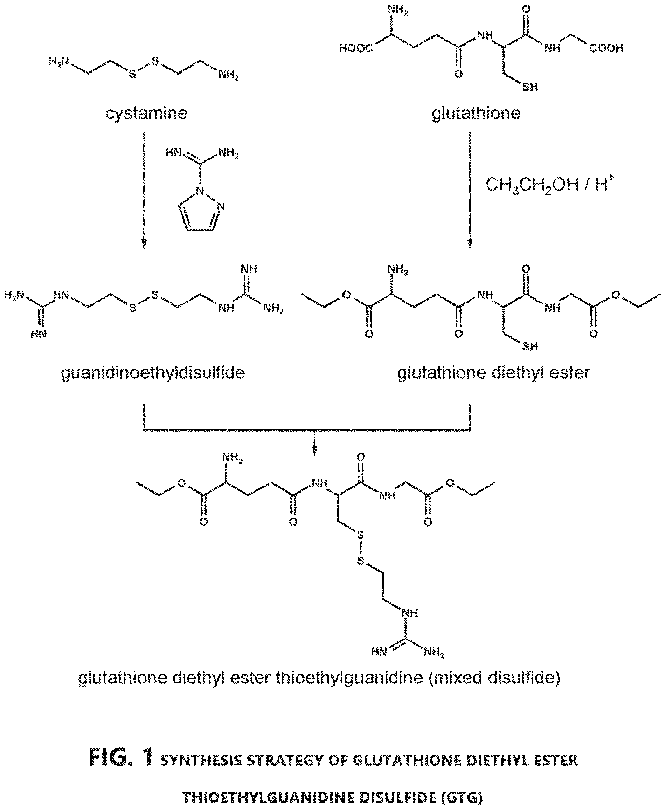

Referring to the synthetic scheme of **FIG.** 1, the synthesis of Glutathione Diethyl Ester Thioethylguanidine Disulfide

### A. Synthesis of Glutathione Diethyl Ester (GSH-OEt₂, 1)

Glutathione Diethyl Ester **(1)** was synthesized following a modified literature protocol (Thornalley, P. K., Esterification of reduced glutathione. Biochem. J. 1991, 275:535-39). Five hundred milligrams of reduced glutathione (1.6 mmole) were dissolved in 10 mL of 0.5 M hydrogen chloride in dry ethanol. The solution was flushed with argon and stirred for 48 h at room temperature. Thereafter, the solvent was removed by vacuum centrifugation to yield the crude product. 1 was purified by preparative HPLC after dissolving about 60 mg of the crude product in 2 ml solvent A. Individual fractions were analyzed by UPLC-MS.

### Preparative High Performance Liquid Chromatography

All preparative runs were carried out with a binary pump (Waters 1525) operating at a flow rate of 15 ml/min. Samples were applied via a 2-ml injection loop (Rheodyne). Separations were carried out at room temperature on an RP C18 column (XBridge Prep C18, 250*19 mm, 5 µm; Waters) connected to a guard column. After the column, 0.3 ml/min was diverted through a valve to a UV-visible detector (Waters 2489), and the rest of the flow (14.7 ml/min) was collected in fractions. The following additional conditions were used for preparative purification of individual compounds: Water (solvent A) and 80% acetonitrile (solvent B (v/v)) were used as eluents. To both solvents, 0.1% trifluoroacetic acid (v/v) was added. Analyses were performed using gradient elution: 10% B (0-5 min) to 20% B (20 min) to 30% B (25 min) to 70% B (35 min) to 100% B (40 to 55 min). The column was equilibrated with 10% B for 15 min prior to the next run. The detection wavelength was set to 230 nm. The fraction size was 14.7 ml.

### UPLC-MS

All chromatographic analyses were carried out in a Waters Acquity UPLC system connected to a Sciex 4500 QTrap (Redwood City, CA). Mass spectrometric analyses were carried out in the multiple reaction monitoring (MRM) mode. Chromatographic separations were carried out in an ACQUITY BEH C18 peptide column (100 * 2.1 mm, 1.7 µm; Waters) connected to a guard column using a flow rate of 0.5 ml/min. Water (solvent A) and acetonitrile (solvent B) were used as eluents. To both solvents, 0.1% heptafluorobutyric acid (v/v) was added. Analyses were performed at a column temperature of 40 °C using gradient elution: 5% B (0-0.25 min) to 20% B (2 min) to 50% B (3 min) to 100% B (3.5-5min). The column was equilibrated at 5% B for 1 min prior to the next analysis. Detection of the analytes was achieved by using full scan mode for the identification of metabolites in preparative HPLC fraction and multiple reaction monitoring for the quantitation in stability experiments. The ion source was run under the following conditions: temperature, 550 °C; ion spray voltage, 4.5 kV; curtain gas, 45 ml/min; nebulizer gas, 60 ml/min; heating gas, 60 ml/min. Mass range for full scan mode was set at 100-700 *m*/*z* and DP was set to 50 V. The MRM parameters are presented in the following table.:

| | | | Quantifier | | | Qualifier 1 | | | Qualifier 2 | | |
|---|---|---|---|---|---|---|---|---|---|---|---|
| | Q1 [*m*/*z*] | DP [V] | Q3 [*m*/*z*] | CE [eV] | CXP [V] | Q3 [*m*/*z*] | CE [eV] | CXP [V] | Q3 [*m*/*z*] | CE [eV] | CXP [V] |
| GTG | 481.2 | 41 | 117.9 | 57 | 8 | 152.0 | 32 | 10 | 205.1 | 35 | 15 |
| GTG - 1*EtOH | 453.1 | 40 | 324.1 | 26 | 7 | 152.0 | 31 | 10 | 118.0 | 35 | 7 |
| GTG - 2*EtOH | 425.1 | 35 | 296.1 | 24 | 13 | 152.0 | 31 | 8 | 118.0 | 31 | 10 |

Fractions containing the product *(m*/*z* 364) were pooled and freeze-dried. **1** was yielded as a colorless solid (TFA salt). NMR spectra (¹H, ¹³C) were according to the literature.

### B. Synthesis of Guanidinoethyl Disulfide / Mercaptoethylguanidine Disulfide (2)

Mercaptoethylguanidine disulfide **(2)** was synthesized following a modified literature protocol (Szabó, C. et al., Pharmacological characterization of guanidinoethyldisulphide (GED), a novel inhibitor of nitric oxide synthase with selectivity towards the inducible isoform. Br. J. Pharmacol. 1996, 118:1659-68). Briefly, 500 mg cystamine dihydrochloride (2.2. mmol) was dissolved in 25 mL water. To this solution 5 g of Amberlite IRA 402 (OH⁻ form) and 0.98 g of 1H-pyrazole-1-carboxamidine HCl (6.6 mmol). The mixture was stirred overnight at room temperature. Afterwards, the reaction mixture was extracted four times with 50 mL ethyl acetate. The aqueous layer was acidified to pH 2 by addition of 2 M hydrochloric acid and freeze-dried. **2** was yielded as a colorless solid (hydrochloride). NMR spectra (¹H, ¹³C) were according to the literature.

### C. Synthesis of Glutathione Diethyl Ester Thioethylguanidine Disulfide (GTG)

One hundred and ten milligrams of **1** (0.23 mmole) and 100 mg **2** (0.32 mmole) were dissolved in 2 mL 0.1 M phosphate buffer containing 1 mM EDTA. The solution was flushed with argon and incubated for 30 min at room temperature. Thereafter, the solution was adjusted to pH 2 with 1 M hydrochloric acid and 2 ml solvent A was added. **3** was purified by preparative HPLC (see below). Individual fractions were analyzed by UPLC-MS. Fractions containing the product (*m*/*z* 481) were pooled and freeze-dried. **3** was yielded as colorless trifluoroacetate salt (40 mg, 0.06 mmole, 26 %). Trifluoroacetic acid was removed by repeatedly dissolving **3** in 0.1 % formic acid and subsequent freeze-drying. ¹H NMR (500 MHz, D₂O with 0.1 % hydrochloric acid): 1.28 (t, J=6.7 Hz, 3H), 1.33 (t, J=6.7 Hz, 3H), 2.28 (m, 2H), 2.62 (m, 2H), 2.98 (m, 2H), 3.02 (m, 1H), 3.22 (m, 1H), 3.58 (t, J=6.6 Hz, 2H), 4.04 (m, 2H), 4.18 (t, J=6.7 Hz, 1H), 4.24 (q, J=6.8 Hz, 2H), 4.34 (q, J=6.6 Hz, 2H), 4.77 (interference by D₂0, confirmed by COSY, 1H). 13C NMR (125 MHz, D₂O with 0.1 % hydrochloric acid): 13.8, 14.8, 26.4, 31.8, 37.1, 39.7, 40.8, 42.6, 53.1, 54.3, 63.7, 64.9, 158.0, 170.7, 172.4, 173.8, 175.3.

### Example 2

### Stability of Glutathione Diethyl Ester Thioethylguanidine Disulfide

To test the stability, GTG was incubated under various conditions. The following buffers were used: 0.1 M phosphate buffer pH 5, 0.1 M phosphate buffer pH 7, 0.1 M phosphate buffer pH 9 and 0.1 % formic acid. 10 µM solutions of GTG in each buffer were stored at 4 °C, 25 °C or 37 °C for up to 48 h, respectively. Sample aliquots were directly injected to ULPC-MS for MRM analysis. For the identification of degradation products sample aliquots were analyzed using full scan mode.

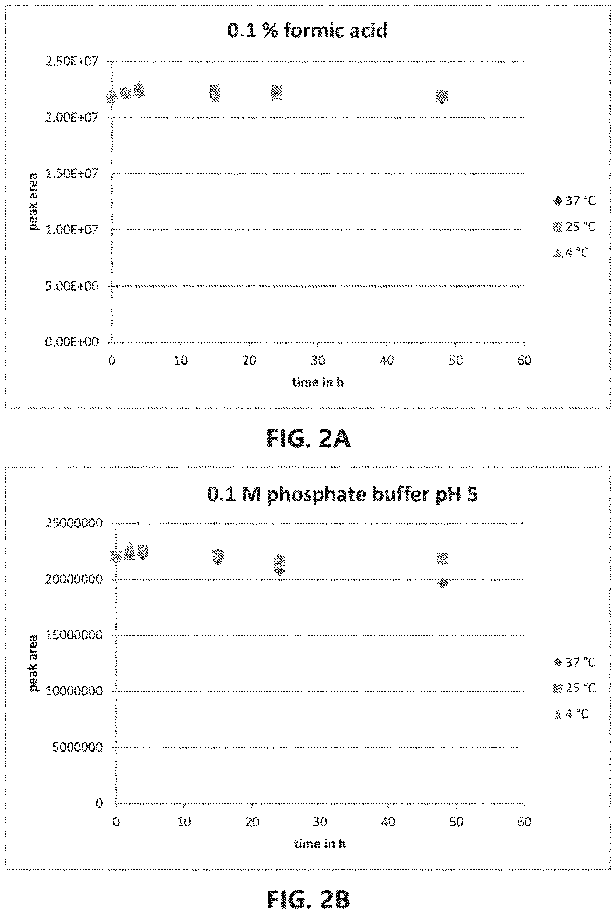

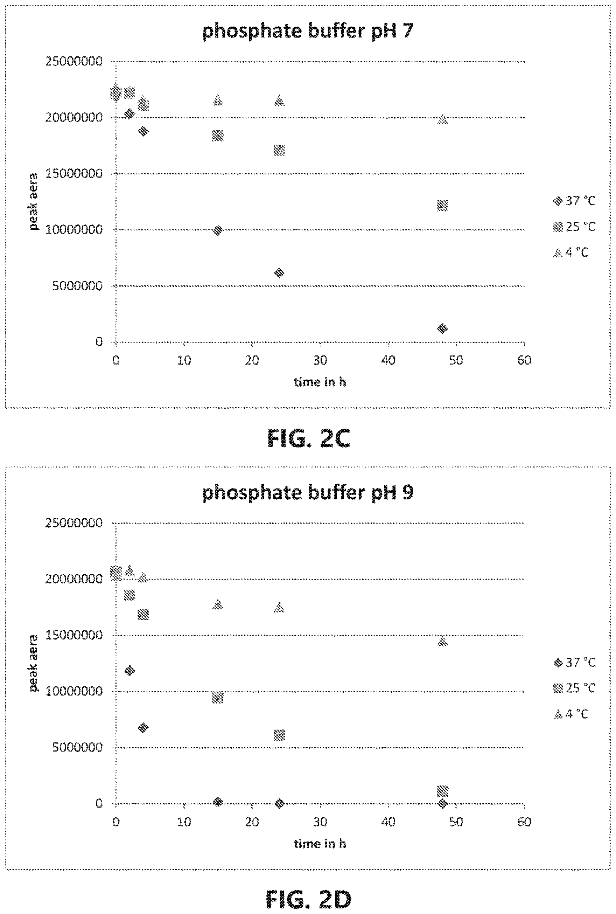

GTG was degraded under most conditions **(****FIGS. 2A-2D****).** Degradation was faster at elevated temperature and high pH. In 0.1 % formic acid GTG was stable at all temperatures. At pH 5 GTG was stable at 4 °C and 25 °C, but slightly degraded at 37 °C. At pH 7 and 9 GTG degraded at all temperatures.

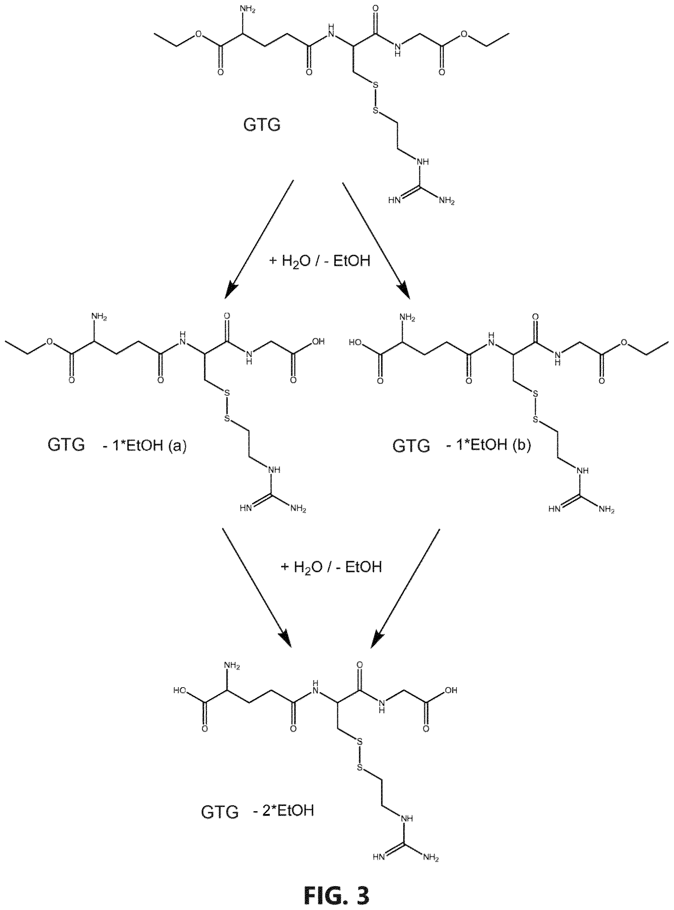

Three degradation products of GTG were identified by mass spectrometry. The dominant degradation pathway was the loss of either one or two molecules of ethanol from both terminal carboxyl groups **(****FIG. 3****).** Therefore, there were two degradation products with the loss of one molecule ethanol (glutamic acid side or glycine side) and one with the loss of two molecules of ethanol (glutamic acid side and glycine side). Both degradation products were monitored in the stability tests (see graph below for 25°C and pH 9). During the incubation, under the conditions described above, reduction of the disulfide bond was not observed.

### Example 3

### Stability of Glutathione Diethyl Ester Thioethylguanidine Disulfide

Diabetes leads to elevated glucose levels and oxidative stress, and the combined effect causes higher levels of reactive carbonyls, such as methylglyoxal, which are precursors for AGEs. The AGE levels are generally higher in tissue and plasma proteins of diabetics than non-diabetics. In addition, the glutathione levels are reduced in diabetes, which may further promote AGE formation. Therefore, we reasoned that a systemic reduction of reactive carbonyls and elevated levels of glutathione could inhibit diabetic complications. We designed, synthesized, isolated, and fully characterized the molecule consisting of glutathione diester and mercaptoethylguanidine; GTG to test simultaneous reduction of reactive carbonyls and elevated levels of glutathione as a treatment or prevention of diabetic complications.

To test the efficacy of GTG in the inhibition of AGE production, αB-Crystallin was incubated with methylglyoxal in the presence or absence of GTG and analyzed for covalent crosslinking and AGE levels. **FIG. 5A** shows Nε-Carboxylethyllysine (CEL) levels in αB-crystallin incubated with GTG and MGO after acid hydrolysis. **FIG. 5B** shows SDS-PAGE analyses of αB-crystallin treated with MGO and GTG. These data demonstrate that GTG significantly reduced covalent crosslinking and AGE formation in αB-crystallin incubated with methylglyoxal.

Next, human lens cells (FHL 124) were incubated with dehydroascorbic acid in the presence or absence of GTG and analyzed for levels of GTG and its metabolites as well as α-dicarbonyls. **FIG. 6A** shows the proposed mechanism for dehydroascorbate (DHA) degradation and formation of reactive α-dicarbonyls and AGEs. **FIG. 6B** shows cellular levels of GSH and mercaptoethylguanidine in FHL 124 cells after treatment with 50 mM GTG for 2h. These data demonstrate that GTG was able to penetrate the plasma membrane of FHL124 cells, and was immediately reduced and hydrolyzed to glutathione and mercaptoethylguanidine. Cells treated for 24h with dehydroascorbic acid and subsequent incubation with GTG showed a significant reduction in reactive α-dicarbonyls derived from dehydroascorbic acid. FHL124 cells were pretreated with GTG before either DHA or MGO was added. **FIG. 6C** shows cellular levels of α-dicarbonyls (threosone, 3-DT, 2,3-DKG, DHA, GO, MGO) and AGEs (CML & CEL) trapped and analyzed as stable quinoxalines.

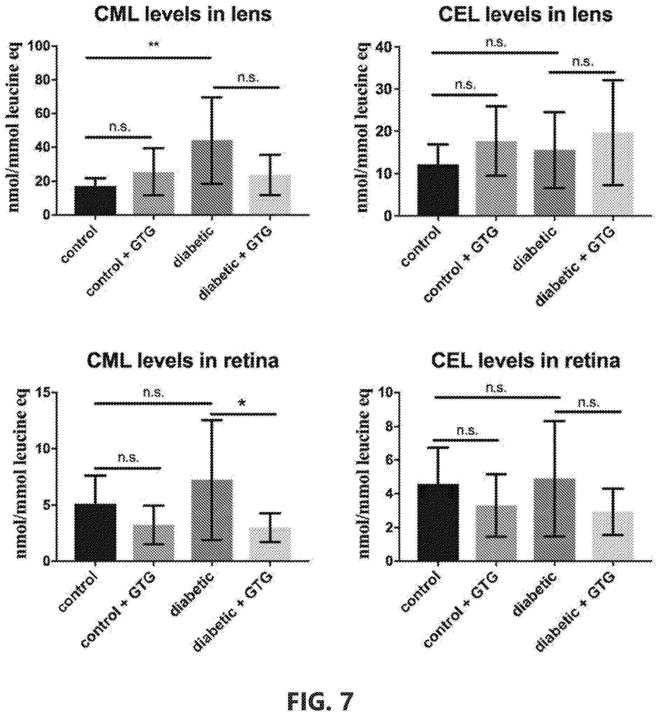

GTG was also tested in diabetic mice. Diabetes was induced by repeated STZ injections. GTG (1 nmole/g body weight) was injected intraperitoneally once every two days. After 8 weeks, lenses and retinas were harvested and analyzed for AGE levels by UPLC-MS/MS. **FIG. 7** shows AGE levels in mice lenses and retinas. GTG (1 nmole/g body weight) was injected i.p. These data demonstrate that when GTG was injected into diabetic animals, AGE levels in the lens and retina were lower when compared to untreated diabetic controls.

Thus, GTG successfully blocks crosslinking and AGE formation in proteins and prevents AGE formation in eye tissues in diabetes.

### Example 4

### GTG is permeable to lens nucleus

GTG was incubated with bovine lenses to demonstrate its entry into the lens nucleus, and its delivery of glutathione (GSH) and mercaptoethylguanidine (MEG). Bovine lenses were incubated with GTG (1.5 mM) for 24 h at 37°C in serum free MEM. Fresh media with 1.5 mM GTG was added after 24 h and the lenses were incubated for another 24 h. After incubation, lenses were shaved from the outer cortex to the nucleus and the shavings were collected in four fractions. The weight of each fraction was noted. To each fraction, the homogenization buffer (PBS, 1.5 mM NEM, pH 7) was added in such a way that the buffer to tissue weight was similar for all fractions. The tissue was subjected to sonication for 30 sec bursts (6 cycles). The samples were then incubated with 1.5 mM n-ethylmaleimide for 2 h at room temperature and subjected to centrifugation at 21,000 g for 20 min at 4°C. The resulting water-soluble fraction was incubated with 10% TCA for 30 min on ice and centrifuged at 21,000 g for 20 min at 4°C. The resulting supernatant was analyzed for GSH and MEG by LC-MS/MS.

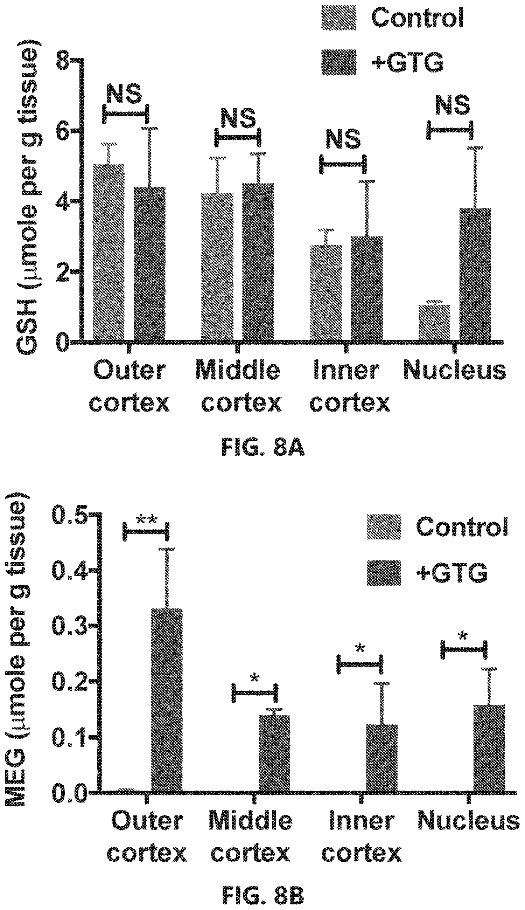

LC-MS/MS analysis of bovine lens fractions (cortex to nucleus) showed that upon incubation with GTG, GSH levels increased in the nucleus of lenses **(****FIG. 8A****);** the GTG treated lenses had GSH levels of 3.79 µmole per gm of tissue, which was higher than (1.05 µmole per gm of tissue) of untreated lenses **(****FIG. 8A****).** GTG treated lenses had MEG levels of 0.33, 0.14, 0.12, 0.16 µmole per gm tissue, respectively in the four fractions, collected from the outer cortex to nucleus **(****FIG. 8B****).** MEG was absent in untreated lenses **(****FIG. 8B****).** These results demonstrate that GTG can penetrate the nucleus of lenses, where it is needed for protecting lenses from oxidative stress. These results also show that upon entering lenses, GTG gets reduced and de-esterified into GSH and MEG, the former can act as an antioxidant and the latter as an anti-glycation agent, the two properties could prevent/reverse presbyopia in aging human lenses.

### Example 5

### GTG reduces lens stiffness

Mouse lenses (6 months old) were incubated with 1.5 mM GTG for 48 h at 37°C in serum free MEM in a CO₂ incubator. Lens stiffness was measured using a set up as described previously (Cheng C, et al., Journal of visualized experiments: JoVE. 2016, (111):10.3791/ 53986). Lens stiffness was measured using a load of 2,700 mg. Axial and equatorial strains were calculated as previously described (Chen, JOVE 2016, supra*).*

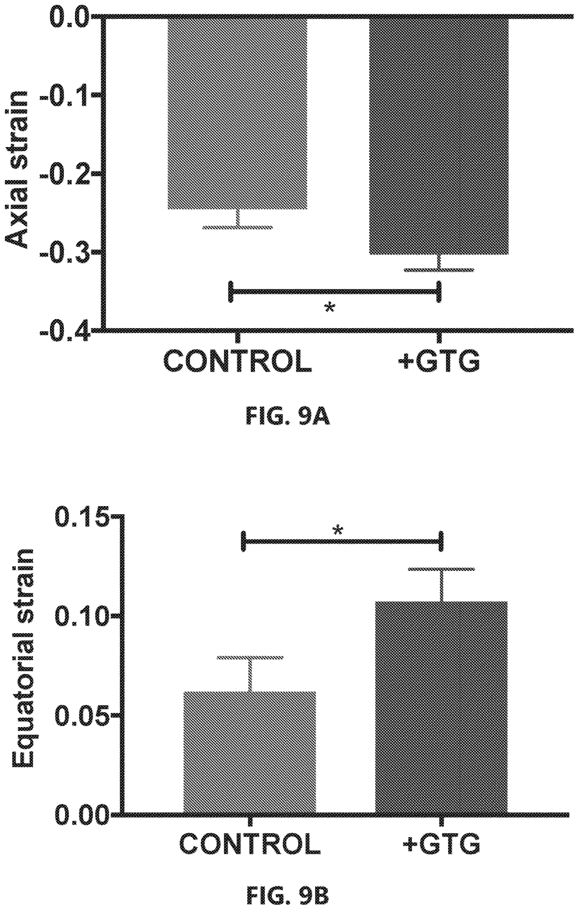

**FIGS. 9A** and **9B** show that GTG treatment improved the axial and equatorial strain by 23% and 72% in comparison to control (untreated) lenses. Thus, GTG reduced lens stiffness. A possible explanation for such an effect could be due to its ability to reduce disulfide crosslinking in lens proteins. Thus, GTG can find use in treating presbyopia.

## Claims

1. A glutathione diethyl ester thioethylguanidine disulfide (GTG) compound having the following chemical structure: 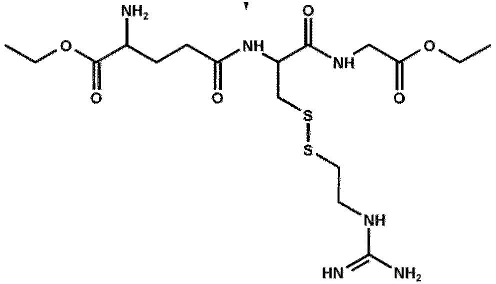 or a pharmaceutically acceptable salt or stereoisomer thereof.

2. A composition comprising the GTG compound of claim 1, or a pharmaceutically acceptable salt or stereoisomer thereof, and a pharmaceutically acceptable excipient.

3. The composition of claim 2, wherein the composition is formulated as a tablet or capsule for oral administration, as a sterile liquid for parenteral administration, or as a liquid for ophthalmic administration.

4. The composition of claim 2, wherein the composition is a mono-phasic pharmaceutical composition suitable for parenteral or oral administration consisting essentially of a therapeutically effective amount of the GTG compound, or a pharmaceutically acceptable salt or stereoisomer thereof, and a pharmaceutically acceptable excipient.

5. A beverage or foodstuff comprising the GTG compound of claim 1, or a pharmaceutically acceptable salt or stereoisomer thereof.

6. A composition comprising the GTG compound of claim 1, or a pharmaceutically acceptable salt or stereoisomer thereof, and a pharmaceutically acceptable excipient for use in the treatment of glycation-related conditions.

7. The composition for use according to claim 6, wherein the composition is formulated as a tablet or capsule for oral administration.

8. The composition for use according to claim 6, wherein the composition is formulated as a sterile liquid for parenteral administration.

9. The composition for use according to claim 6, wherein the composition is formulated as a liquid for ophthalmic administration.

10. The composition for use according to claim 6, wherein the composition is a beverage or foodstuff.

11. The composition for use according to claim 6, wherein the glycation-related conditions comprise formation of glycation end products, formation of advanced glycation end products (AGEs), glycation reactions of proteins, lipids and/or nucleic acids, aging effects related to glycation reactions, cancer, diabetes, complications of diabetes (Type I and II), rheumatoid arthritis, Alzheimer's disease, uremia, neurotoxicity, atherosclerosis, inflammatory reactions, ventricular hypertrophy, angiopathy, myocarditis, nephritis, arthritis, glomerulonephritis, microangiopathies, and renal insufficiency.

12. A composition comprising the GTG compound of claim 1, or a pharmaceutically acceptable salt or stereoisomer thereof, and a pharmaceutically acceptable excipient for use in treating, preventing, ameliorating, or reducing the progression of presbyopia.

13. The composition for use according to claim 12, wherein the composition is formulated as a tablet or capsule for oral administration, as a sterile liquid for parenteral administration, or as a liquid for ophthalmic administration.

14. The composition for use according to claim 12, wherein the composition is a beverage or foodstuff.

15. The composition for use according to claim 12, wherein reducing the progression of presbyopia includes reducing crosslinking of lens proteins.

## Patentansprüche

1. Glutathiondiethylester-Thioethylguanidindisulfid-Verbindung (GTG), die die folgende chemische Struktur aufweist: 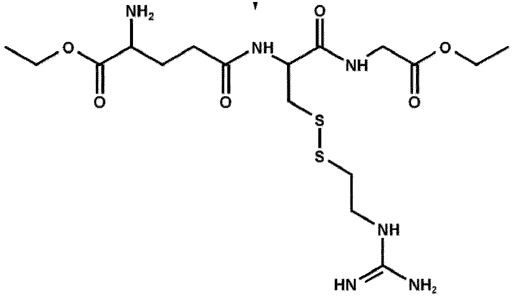 oder ein pharmazeutisch akzeptables Salz oder Stereoisomer davon.

2. Zusammensetzung, umfassend die GTG-Verbindung nach Anspruch 1 oder ein pharmazeutisch akzeptables Salz oder Stereoisomer davon und einen pharmazeutisch akzeptablen Arzneimittelträger.

3. Zusammensetzung nach Anspruch 2, wobei die Zusammensetzung als eine Tablette oder Kapsel zur oralen Verabreichung, als eine sterile Flüssigkeit zur parenteralen Verabreichung oder als eine Flüssigkeit zur ophthalmischen Verabreichung formuliert ist.

4. Zusammensetzung nach Anspruch 2, wobei die Zusammensetzung eine einphasige pharmazeutische Zusammensetzung ist, die zur parenteralen oder oralen Verabreichung geeignet ist, im Wesentlichen bestehend aus einer therapeutisch wirksamen Menge der GTG-Verbindung oder einem pharmazeutisch akzeptablen Salz oder Stereoisomer davon und einem pharmazeutisch akzeptablen Arzneimittelträger.

5. Getränk oder Nahrungsmittel, das die GTG-Verbindung nach Anspruch 1 oder ein pharmazeutisch akzeptables Salz oder Stereoisomer davon umfasst.

6. Zusammensetzung, die die GTG-Verbindung nach Anspruch 1 oder ein pharmazeutisch akzeptables Salz oder Stereoisomer davon und einen pharmazeutisch akzeptablen Arzneimittelträger zur Verwendung bei der Behandlung von mit Glykation in Zusammenhang stehenden Zuständen umfasst.

7. Zusammensetzung zur Verwendung nach Anspruch 6, wobei die Zusammensetzung als eine Tablette oder Kapsel zur oralen Verabreichung formuliert ist.

8. Zusammensetzung zur Verwendung nach Anspruch 6, wobei die Zusammensetzung als eine sterile Flüssigkeit zur parenteralen Verabreichung formuliert ist.

9. Zusammensetzung zur Verwendung nach Anspruch 6, wobei die Zusammensetzung als eine Flüssigkeit zur ophthalmologischen Verabreichung formuliert ist.

10. Zusammensetzung zur Verwendung nach Anspruch 6, wobei die Zusammensetzung ein Getränk oder Nahrungsmittel ist.

11. Zusammensetzung zur Verwendung nach Anspruch 6, wobei die mit Glykation in Zusammenhang stehenden Zustände die Bildung von Glykationsendprodukten, die Bildung von fortgeschrittenen Glykationsendprodukten (AGEs), Glykationsreaktionen von Proteinen, Lipiden und/oder Nukleinsäuren, Alterungseffekte im Zusammenhang mit Glykationsreaktionen, Krebs, Diabetes, Komplikationen von Diabetes (Typ I und II), rheumatoide Arthritis, Alzheimer-Krankheit, Urämie, Neurotoxizität, Arteriosklerose, entzündliche Reaktionen, ventrikuläre Hypertrophie, Angiopathie, Myokarditis, Nephritis, Arthritis, Glomerulonephritis, Mikroangiopathien und Niereninsuffizienz umfassen.

12. Zusammensetzung, die die GTG-Verbindung nach Anspruch 1 oder ein pharmazeutisch akzeptables Salz oder Stereoisomer davon und einen pharmazeutisch akzeptablen Arzneimittelträger zur Verwendung beim Behandeln, Vorbeugen, Lindern oder Verringern des Fortschreitens von Alterssichtigkeit umfasst.

13. Zusammensetzung zur Verwendung nach Anspruch 12, wobei die Zusammensetzung als eine Tablette oder Kapsel zur oralen Verabreichung, als eine sterile Flüssigkeit zur parenteralen Verabreichung oder als eine Flüssigkeit zur ophthalmischen Verabreichung formuliert ist.

14. Zusammensetzung zur Verwendung nach Anspruch 12, wobei die Zusammensetzung ein Getränk oder Nahrungsmittel ist.

15. Zusammensetzung zur Verwendung nach Anspruch 12, wobei Verringern des Fortschreitens von Alterssichtigkeit Verringern einer Vernetzung von Linsenproteinen beinhaltet.

## Revendications

1. Composé de disulfure de thioéthylguanidine d'ester diéthylique de glutathion (GTG) présentant la structure chimique suivante : 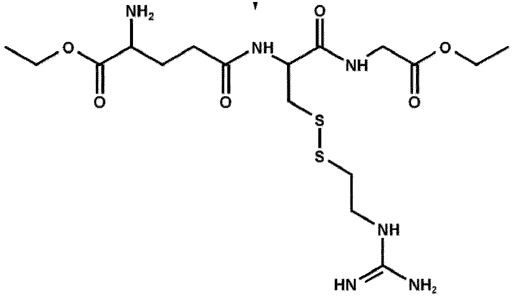 ou sel ou stéréoisomère pharmaceutiquement acceptable de celui-ci.

2. Composition comprenant le composé GTG selon la revendication 1, ou un sel ou un stéréoisomère pharmaceutiquement acceptable de celui-ci, et un excipient pharmaceutiquement acceptable.

3. Composition selon la revendication 2, dans laquelle la composition est formulée sous la forme d'un comprimé ou d'une capsule pour une administration orale, sous la forme d'un liquide stérile pour une administration parentérale, ou sous la forme d'un liquide pour une administration ophtalmique.

4. Composition selon la revendication 2, dans laquelle la composition est une composition pharmaceutique monophasique appropriée pour une administration parentérale ou orale se composant essentiellement d'une quantité thérapeutiquement efficace du composé GTG, ou d'un sel ou stéréoisomère pharmaceutiquement acceptable de celui-ci, et d'un excipient pharmaceutiquement acceptable.

5. Boisson ou produit alimentaire comprenant le composé GTG selon la revendication 1, ou un sel ou stéréoisomère pharmaceutiquement acceptable de celui-ci.

6. Composition comprenant le composé GTG selon la revendication 1, ou un sel ou un stéréoisomère pharmaceutiquement acceptable de celui-ci, et un excipient pharmaceutiquement acceptable pour une utilisation dans le traitement d'affections liées à la glycation.

7. Composition pour une utilisation selon la revendication 6, dans laquelle la composition est formulée sous la forme d'un comprimé ou d'une capsule pour une administration orale.

8. Composition pour une utilisation selon la revendication 6, dans laquelle la composition est formulée sous la forme d'un liquide stérile pour une administration parentérale.

9. Composition pour une utilisation selon la revendication 6, dans laquelle la composition est formulée sous la forme d'un liquide pour une administration ophtalmique.

10. Composition pour une utilisation selon la revendication 6, dans laquelle la composition est une boisson ou un produit alimentaire.

11. Composition pour une utilisation selon la revendication 6, dans laquelle les affections liées à la glycation comprennent la formation de produits finaux de glycation, la formation de produits finaux de glycation avancée (AGE), des réactions de glycation de protéines, de lipides et/ou d'acides nucléiques, des effets du vieillissement liés à des réactions de glycation, le cancer, le diabète, des complications du diabète (type I et II), la polyarthrite rhumatoïde, la maladie d'Alzheimer, l'urémie, la neurotoxicité, l'athérosclérose, des réactions inflammatoires, l'hypertrophie ventriculaire, l'angiopathie, la myocardite, la néphrite, l'arthrite, la glomérulonéphrite, les microangiopathies et l'insuffisance rénale.

12. Composition comprenant le composé GTG selon la revendication 1, ou un sel ou un stéréoisomère pharmaceutiquement acceptable de celui-ci, et un excipient pharmaceutiquement acceptable pour une utilisation dans le traitement, la prévention, l'amélioration ou la réduction de la progression de la presbytie.

13. Composition pour une utilisation selon la revendication 12, dans laquelle la composition est formulée sous la forme d'un comprimé ou d'une capsule pour une administration orale, sous la forme d'un liquide stérile pour une administration parentérale, ou sous la forme d'un liquide pour une administration ophtalmique.

14. Composition pour une utilisation selon la revendication 12, dans laquelle la composition est une boisson ou un produit alimentaire.

15. Composition pour une utilisation selon la revendication 12, dans laquelle la réduction de la progression de la presbytie inclut la réduction de la réticulation de protéines du cristallin.
